# EUROPEAN PATENT SPECIFICATION

(11) **EP 1 622 673 B1**
(45) Date of publication and mention of the grant of the patent: **17.12.2008**
(21) Application number: 04759871.9
(22) Date of filing: 15.04.2004
(51) Int. Cl.: A61M 31/00, A61K 48/00, A61M 5/142, A61M 5/172, A61B 5/0205, A61N 1/362

(54) **SYSTEM FOR THE DELIVERY OF A BIOLOGIC THERAPY WITH DEVICE MONITORING AND BACK-UP**
SYSTEM ZUR VERABREICHUNG EINER BIOLOGISCHEN THERAPIE MIT ÜBERWACHUNG UND BACK-UP
SYSTEME POUR LA CONDUITE DE THERAPIE BIOLOGIQUE AVEC CONTROLE DE DISPOSITIF ET PROCESSUS DE RESERVE

(30) Priority: 23.04.2003 US 464767 P
(43) Date of publication of application: 08.02.2006
(73) Proprietor: MEDTRONIC, INC., Minneapolis MN 55432-5604 (US)
(72) Inventor: LASKE, Timothy, G., Shoreview, MN 55126 (US); SIGG, Daniel, C., St. Paul, MN 55108 (US); SOYKAN, Orhan, Shoreview, MN 55126 (US)
(74) Representative: Hughes, Andrea Michelle
(86) International application number: PCT/US2004/011581
(87) International publication number: WO 2004/093969

(56) References cited:
- WO-A-02/28470
- WO-A-99/39624
- US-A- 4 003 379
- US-A- 6 155 267
- US-A1- 2003 009 145
- US-A1- 2003 032 998
- US-B1- 6 224 566

## Description

The present invention relates to cardiac therapies and more specifically to the delivery, monitoring and back-up of biologic therapies with device-based therapies.

Traditionally, various cardiac arrhythmias have been managed by drug therapies, device therapies, or a combination of the two. For example, various implantable medical devices (IMD's) such as, implantable pulse generators (IPG's), pacemakers, cardioverters, defibrillators (ICD), or the like can be implanted and deliver electrical stimulation to the heart to provide various pacing or shocking functions. With such a device, a can or device housing is implanted subcutaneously with one or more leads extending to an appropriate location within, or external to, the heart. The therapy is generated within the can and transmitted along the lead to an electrode affixed to the heart tissue.

Various drug therapies can also be employed to manage cardiac conditions. Often, a particular drug therapy may be utilized in conjunction with a given IMD so that the two therapies supplement one another.

Typical examples of such devices are disclosed in US patents US-A-4 003 379 and US-A-2003/0032998.

Recently, the concept of introducing a biologic therapy (e.g., gene therapy) has shown substantial promise. That is, various agents are introduced into the tissue to achieve a desired result. For example, biologic pacing can be achieved by either introducing new pacing cells or altering the chemical structure of existing cells to create or modify a pacing or nodal function. While intriguing, the introduction of biologics is difficult to control. That is, the result of the therapy is difficult to predict. A spectrum of possible results includes successful modification of a region of tissue, partially successful modification, errant modification, or no modification at all. Furthermore, unlike device-based therapies, the resulting benefit is not instantaneous; rather, the results (positive or negative) take time to achieve. Often, this time frame is on the order of weeks and possible months. In the meantime, the effect on the desired organism (e.g., cardiac tissue) is uncertain and the underling condition that prompted the therapy still poses a risk.

The present invention provides a system as defined in claim 1.

Preferred embodiments will now be described by way of example only, with reference to the drawings.
FIG. 1 is a schematic illustration of an IMD with a lead placed into the right atrium (RA) of a heart and biologic reservoir coupled with the lead.
FIG. 2A is a schematic side, elevational view of a portion of a lead including a solution delivering lumen.
FIG. 2B is a top, planar view of the lead of FIG. 2A.
FIG. 3 is a flow chart illustrating the possible actions taken by the IMD.
FIG. 4 is a schematic illustration of an IMD along with an implantable biologic reservoir operatively coupled with the IMD.
FIG. 5 is a schematic illustration of a heart and the associated electrical depolarization processes.
FIG. 6 is a schematic illustration of ionic currents during the depolarization and repolarization of a cardiomyocyte.
FIG. 7 is a schematic illustration of the genes governing cardiac ion currents.
FIG. 8 is a schematic illustration of an IMD having an injection port.

The device according to present invention, in one embodiment, provides a potentially curative therapy for certain cardiac conditions that utilizes a systems approach. The systems approach incorporates the introduction of a biologic or drug, the introduction of a device-based back-up, and the introduction of the capability to terminate the biologic function.

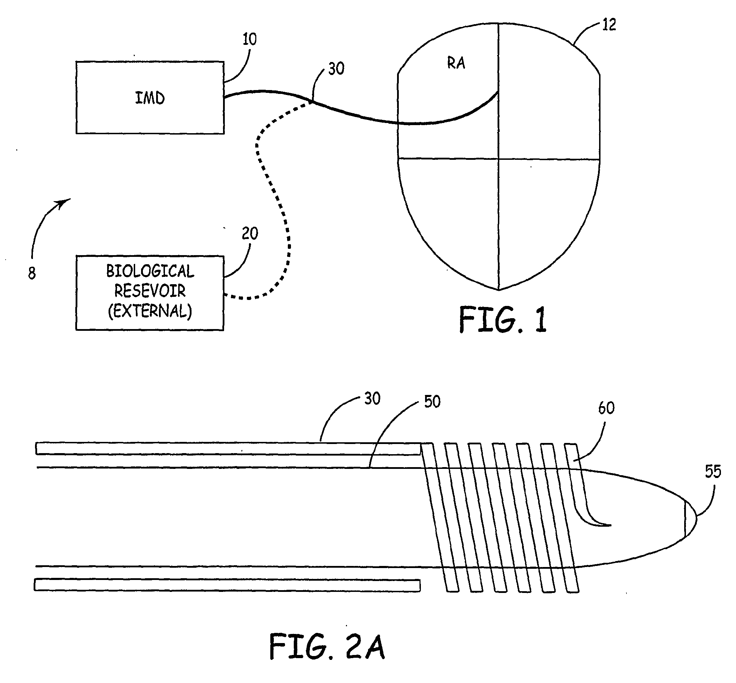

For example, FIG. 1 illustrates a system 8. System 8 will be described with reference to the specific treatment of common exemplary cardiac arrhythmias originating in the AV-node or SA node such as AV-block, sick sinus syndrome, atrial tachycardias, etc.; however, it will be appreciated that the system 8 can be utilized to treat a variety of cardiac conditions, including heart failure, as well as neurological conditions, cancer, and provide islet cell transplantation, or other cell/gene therapies. For example, the present biologic therapy delivery management system 8 is useful to, among other things, provide curative therapy for cardiac arrhythmias, generating biologic pacemakers, performing AV-nodal conduction modulation (e.g., reducing conduction velocities in the AV-node in atrial fibrillation), and modifying focal tissue for tachycardias. FIG. 1 schematically illustrates how an IMD 10 is implanted and coupled with a heart 12. Specifically, IMD 10 may be an IPG to provide a pacing function, an ICD to provide shock, or a device including any combination of these functions. A lead 30 is coupled to the IMD 10 and is placed appropriately within the heart 12. The lead 30 terminates in an appropriate electrode or sensor to deliver the appropriate therapy and, if desired, to monitor the appropriate variables.

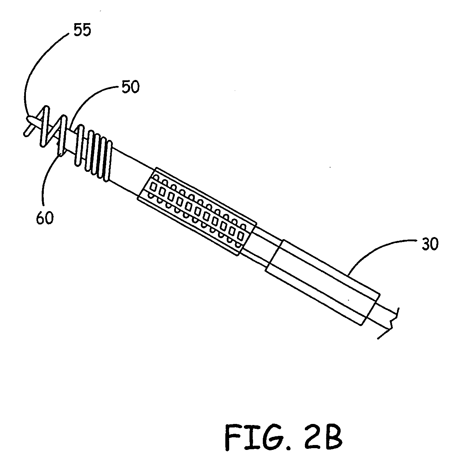

Also provided is a biologic reservoir 20 containing the desired biologic agent and possibly other agents to either supplement or terminate the biologic, as will be described in greater detail. Referring to FIG. 1, 2A and 2B, a stylet or lumen 50 interconnects the reservoir 20 with the myocardium in the right atrium (RA), and more specifically in this example, the AV or SA node. The lumen 50 is guided through the lead 30 and may either be removed after the procedure or left in place. The combination of the lead 30 and lumen 50 is capable of performing a variety of functions. The lumen 50 includes a distal tip 55. Distal tip 55 may be forced into contact with the myocardial tissue or forced into the myocardial tissue thereby permitting delivery of the biologic or other agent through the lumen 50 and into or onto the tissue. An anchoring mechanism 60 may be attached to the lead 30 to facilitate the attachment of the lead 40 to the myocardial tissue. Anchoring mechanism 60 may, for example, take the form of a helical coil that can be rotationally advanced through an appropriate depth of tissue. In this manner, the lead 30 is secured to a targeted area and the lumen 50 can be advanced, allowing for the delivery of the biologic. In addition, the anchoring mechanism 60 can function as an electrode to deliver various therapies, sense certain parameters, or provide for ablation of the surrounding tissue, as will be described more fully below. Alternatively, an electrode separate from the anchoring mechanism may be provided. One lead structure suitable for use as lead 30 and lumen 50 is more fully described in US patent number 7 103 418, entitled "Active Fluid Delivery Catheter" by the same applicants.

In use, a patient suffering from a particular condition is designated to receive the combined biologic and device therapy. The lead 30 is delivered into the heart, e.g., the RA and the appropriate position is targeted. Lead positioning would be done by using one or more of the various mapping techniques such as electrophysiologic, radiologic, ultrasound echographic or MRI-quided. For example, the tip of the lead 30 may be advanced to contact the AV node, SA node, or other desired location. After the lead 30 is properly positioned, it is rotated; thereby securing the helical anchor 60 into the myocardial tissue. Thus, the lead 30 is now positioned and secured within the heart, and particularly in the proper location within the RA.

The lumen 50 is then inserted into the lead 30 (or simply advanced if already present) until the tip 55 is proximate the myocardial tissue. Then, the tip 55 is either advanced to contact the myocardial tissue or to penetrate therethrough, depending upon the nature of the biologic that will be delivered. If not already coupled, a proximal end of the lumen 50 is coupled with a biologic reservoir 20 external to the patient. The biologic is delivered from the reservoir 20 into the myocardial tissue. Typically, the biologic is delivered as a solution into the tissue. Once delivery is complete, the proximal end of the lumen 50 is disconnected from the reservoir and the lumen 50 is either removed from the lead 30 or seated for storage within the lead 30.

The IMD 10 is coupled with the lead 30 that is implanted in a patient. In this embodiment, the lead provides a dual function as a conduit for the delivering of a biologic or other drug agent also as a means for cardioverting, defibrillating and/or pacing. In-one embodiment, IMD 10 includes cardiac monitoring features that monitor various cardiac parameters. In this manner, IMD 10 can determine the effectiveness of the delivered biologic. As previously explained, the biologic will take time to reach efficacy. Thus, in the meantime, IMD 10, via lead 30 can also provide an appropriate pacing, cardioversion and/or defibrillation therapy.

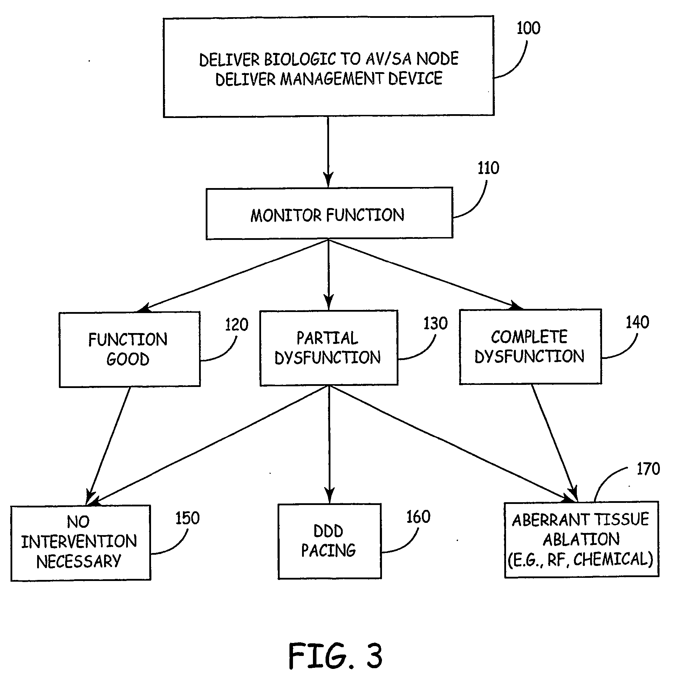

FIG. 3 is a flowchart illustrating the system 8 parameters. As previously explained, the IMD 10 is implanted and the biologic is delivered (100). The IMD 10 monitors (110) cardiac function to determine the efficacy of the biologic over time. The IMD 10 serves at least two therapy roles. Specifically, since the biologic requires time to act. Cardiac functioning may be impaired as a result of the underlying cardiac dysfunction. Thus, the IMD 10 may deliver therapy during this time; however, this does not indicate a failure of the biologic. Alternatively, after a period of time the biologic will have either successfully altered the cell structure and positively affected the cardiac parameter (e.g., reformed a node or generated pacing cells). In such a case, the monitored cardiac performance is good (120). Once such a state is confirmed, IMD 10 will not need to deliver subsequent therapy 150. However, IMD 10 will continue to monitor and be available to deliver therapy in the event the biologic function is subsequently impaired or diminished.

Even when the biologic is successful or partially successful, the IMD 10 may provide pacing therapy in some cases. For example, if the patient has an episode of atrial fibrillation or flutter, the IMD 10 may provide overdrive pacing to control or terminate the condition.

As another possibility, the biologic may improve cardiac performance to some extent or otherwise provide a change in condition, but some continued dysfunction may remain (130). For example, a new SA node may be formed, but without a rate response. In such a case, the IMD 10 will take the appropriate therapeutic action, depending upon the measured parameters. If the dysfunction is tolerable, no intervention need be taken (150). If pacing or a similar therapy is required, that therapy is delivered (160).

Alternatively, the situation may warrant the termination of the biologic 170. This decision tree can be programmed into the IMD 10 or the IMD 10 can provide the monitored data to an external source and the appropriate course of action can be externally programmed into the IMD 10.

The IMD 10 may determine that the biologic has completely failed (140) either by achieving no improvement or by possibly generating aberrant tissue. In such a case, the biologic may be destroyed and other therapies may be employed, such as overdrive pacing. One mechanism to destroy the biologic is to use the lead 30 to ablate the surrounding tissue by delivering an appropriate electrical current, thereby destroying the biologic and the tissue that was generated. Alternative methods of ablation could be used such as RF or chemical delivery, delivered via the lead 30 or by external means. In one embodiment, the lumen 50 (either because it is still in place or reinserted through lead 30) is used to deliver a cytotoxic agent to the target area thereby destroying the tissue affected by the biologic.

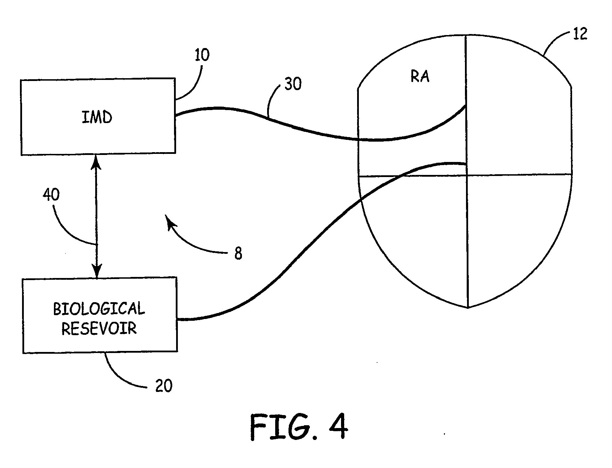

If.the efficacy of the biologic is less than optimal or even completely dysfunctional, the same or alternative biologics could be reintroduced to reattempt the therapy. In one embodiment, the proximal end of the lead 30 is re-exposed and reconnected to reservoir 20 (or the like) to deliver new or additional biologics. The cytotoxic chemical could also be introduced in this fashion. Once the lead 30 is re-exposed the lumen 50 can be accessed if present or inserted for use. FIG. 4 illustrates another embodiment where biologic reservoir 20 is implanted subcutaneously along with the IMD 10. A second lead or lumen 32 is illustrated to allow fluid delivery from the reservoir 20, which includes a pumping mechanism, to the targeted cardiac tissue. It should be appreciated that the lumen 32 may be a separate component, as illustrated, or could function as lumen 50 and proceed within lead 30 as previously described. With biologic reservoir 20 implanted, the biologic could be delivered over time, redelivered to reinitiate or restart therapy, or by providing a separate fluid chamber, automatically deliver a cytotoxic agent to terminate the biologic. By forming an appropriate connection 40 with IMD 10, biologic reservoir 20 can be triggered by the IMD to take the appropriate course of action.

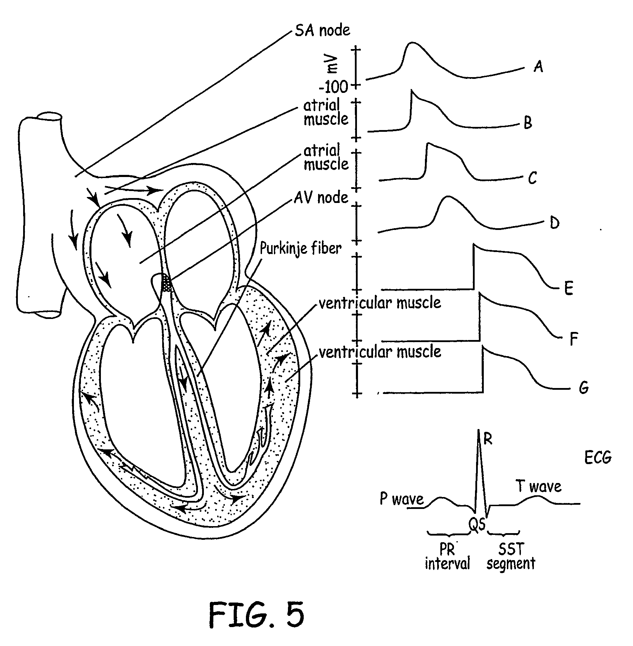

FIG. 5 illustrates the functions of the SA and AV node, as well as how their pathologies differ. The basic electrophysiology of the cardiac muscle and the cardiac nodes is presented. A goal of the therapy with the present system 8 is to restore certain pathologic conditions back to forms as close as possible to the ones shown.

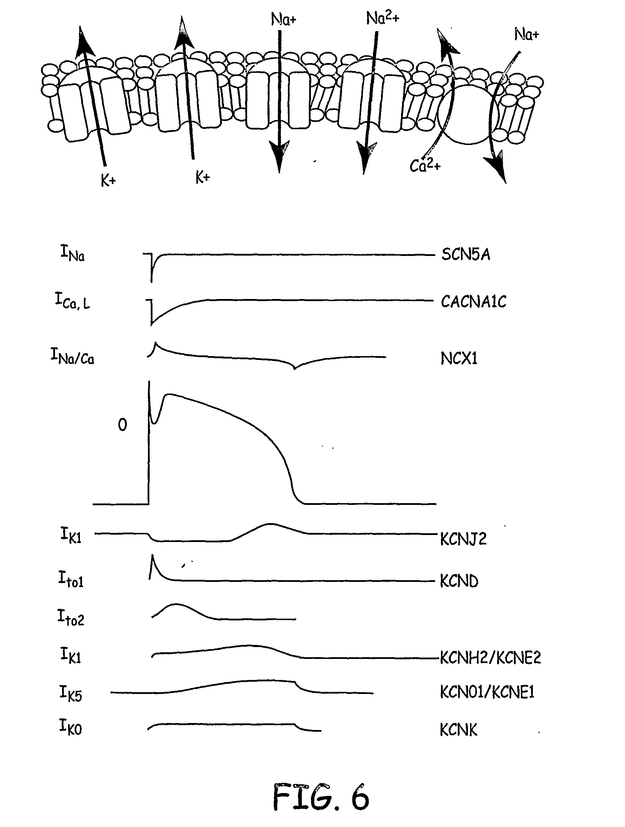

The present invention is applicable to many cardiac and neurological conditions. In some embodiments, biologics are used to act on cardiac conduction pathways. FIG. 6 illustrates the basic electrophysiology of the cardiac muscle and the cardiac nodes. The electrophysiology of a cardiomyocyte is governed by the flow of ions across the cell membrane and across the membranes of the intracellular organelles, such as the SR and the mitochondria. Flow of these ions across the membranes are not constant, but vary in time and morphology, as illustrated. Pathologies distorting these currents would affect the electrophysiology of the cells, as well as the entire organ. For example, a defective ion channel might cause a cell to depolarize prematurely and initiate conduction of the signals with wrong timing, where gene therapy could be used to correct the abnormal channel function.

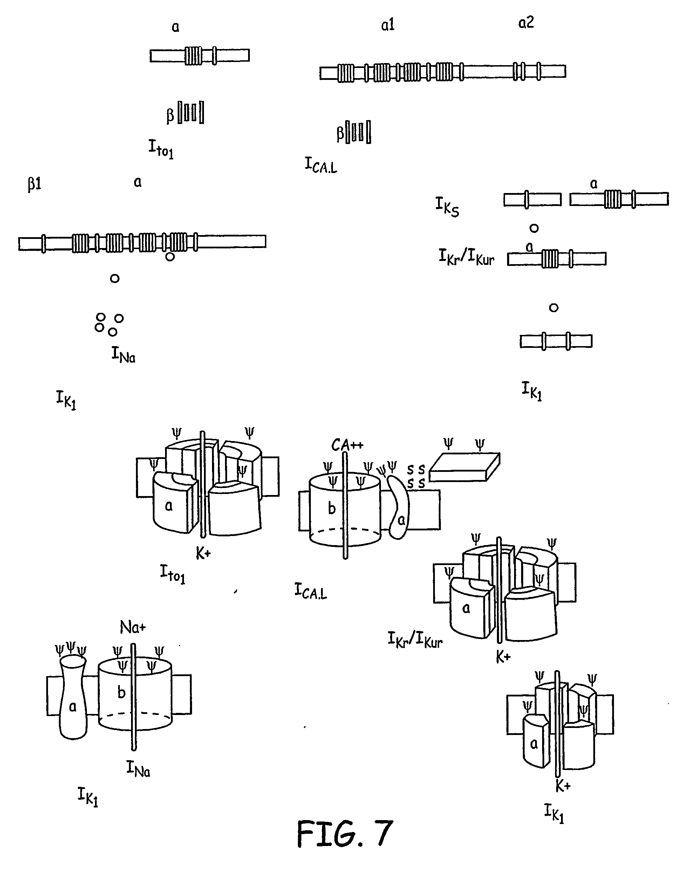

All the currents shown in FIG. 6 are governed by channel proteins, which are coded by genes, which are diagrammed in FIG 7. Genetic therapies delivered by the system 8 can be enhancing, reducing the function of the genes responsible for the electrophysiology, or can deliver genes that mimic cardiac pacemaker potentials (e.g., slow diastolic depolarization) derived from other organ systems (e.g. the brain). Genetic therapies for the enhancement of the gene expression can be via: over expression of the gene, over expression of a promoter, under expression of a silencer, over expression of a regulatory, over-expression of auxiliary subunits responsible for the pacemaker potentials. Genetic therapies for the reduction of the gene expression can be via: RNA interference (e.g. siRNA), RNA silencing (missense), over-expression of suppressor elements, blockade of transcription by decoy technologies, dominant negative suppression using a mutant channel gene. Genetic therapies can be delivered via: viral vectors such as retrovirus, adenovirus, adeno-associated virus, non-viral vectors including, plasmids, lipid based, via-electroporation (from the delivery lead itself), or genetically engineered cells (with pacemaker activity and conductivity).

Cellular therapies may consist of autologous cells (cultured, altered, or *ex-vivo* transfected) including: fibroblasts, bone marrow derived stem cells, skeletal muscle derived, or cardiac derived - SA nodal cells. Cellular therapies may also include allogeneic cells, such as mesenchymal stem cells, or xenogeneic cells.

Cells that are placed into the myocardium would act as new conduction pathways, new sinus nodes, new insulators to break or slow down the signals, and/or new AV nodes. Overall, the new biological node would create new functions to replace the lost ones, create blockage of pathways and/or reduce local conduction velocities in tissues (myocardial and conduction system).

In addition to the cellular modifications, the IMD 10 would provide the monitoring and necessary intervention, such as pacing, burst pacing, bias voltages to modify local potentials, and high energy shocks. In addition, the IMD 10 could provide an alarm function for notifying a physician and/or patient of aberrant tissue function. This function can use transtelephonic or telemetered data transmission protocols.

In certain embodiments, the lead system provides a platform for the initial delivery of the biologics and/or drugs as well as the re-intervention for additional delivery or secondary therapy. The lead 30 also provides electrical conduction for monitoring functions such as monophasic action potentials, action potential durations, depolarization frequency (heart rate, atrial rate, ventricular rate), and QT, ST, QRS, and P wave morphologies. Further, the lead 30 allows for intervention such as overdrive pacing or the delivery of shocks to terminate arrhythmias and provides a route for ablation. Ablation techniques could include RF energy, alcohol, or other ablation technologies.

While the foregoing has been described with respect to the introduction of a biologic into the heart, it should be appreciated that various fluids and substances having a wide variety of purposes can be introduced into the heart in this manner. Many types of drugs (e.g., amiodarone), proteins (e.g., MMP-9 (matrix metallo protease) therapeutic use for heart failure), anti-arrhythmic compounds, and other therapeutic solutions can be delivered in various doses and directly to a target area. This increases the potency and the efficacy, as the delivery is local. Furthermore, fluids and even substances can be withdrawn from the heart 12, out through the lumen 50.

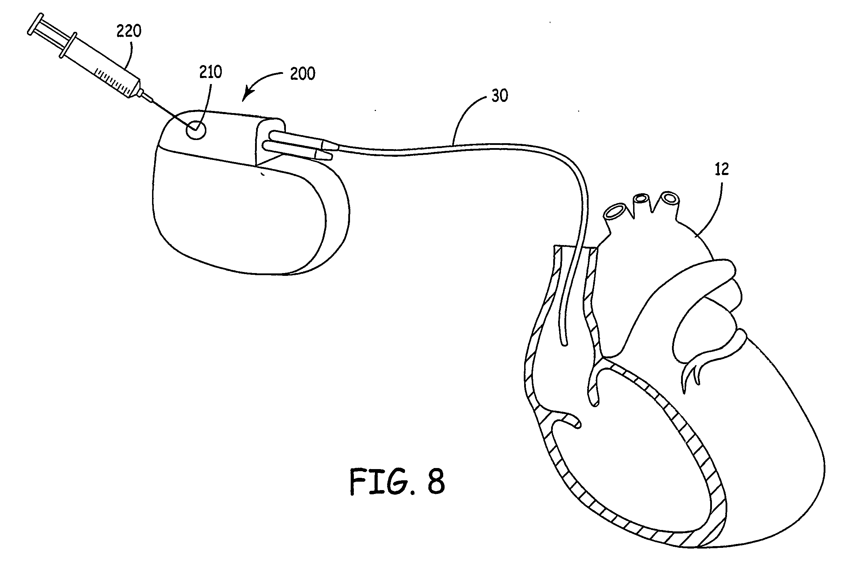

As previously described, it is possible to implant a reservoir 20 so that the biologic or other solutions are selectively deliverable. The reservoir 20 could be implanted with a single useable quantity or it could be externally refillable. FIG. 8 illustrates another embodiment where a more traditional IMD 200 (e.g., a pacemaker, ICD or the like) includes the lead 30 having a fluid delivering lumen 50 as previously described. The IMD 200 includes a fluid access port 21 that is in fluid communication with lead 30. In this way, the fluid access port 210 can be accessed subcutaneously after implantation by inserting a syringe through the skin and piercing the fluid access port 210. The fluid access port includes a self sealing membrane that will automatically reseal after the needle 220 is withdrawn.

In this manner, fluids can be delivered from the syringe to the target area of the heart through the lead 30 lumen 50 combination. That is, advancing the piston of the syringe 220 generates sufficient pressure to transfer the contents of the syringe 220, through the lumen 50 and into the heart 12. This would be useful for the introduction of a biologic as explained above as well as for introducing various drugs or compounds for any number of purposes. In addition, the syringe or a similar device can be used to withdraw fluid (e.g., from the interstatial space) from the heart for therapeutic purposes or for testing and evaluation. For example, the withdrawn samples could be used to assess inflammation, transplant rejection, infection or for other diagnostic purposes.

Other mechanism can be employed to deliver fluids to the target area after implantation. For example, a transvascular catheter could be advanced within the coronary vasculature. Epicardial access could be obtained through surgical ports (e.g., a thoracotomy). Alternatively, endocardial catheter could be guided by intracardial EGM and/or other mapping modalities.

The present invention has been described with reference to certain embodiments useful in cardiac applications. It should be appreciated that the present invention is not so limited and may be utilized in various portions of the body to affect various organs, tissue, systems, anatomical features, or physiological functions including, for example, the heart, brain, pancreas, liver, stomach, venous system, nervous system, or spine. Furthermore, it should be appreciated that the present invention may be utilized to deliver therapies or treatments that affect disparate or remote organs or systems. For example, biologics may be introduced in one site that affect a nervous pathway or function that ultimately affects or controls a remote physiological function.

While multiple embodiments are disclosed, still other embodiments of the present invention will become apparent to those skilled in the art from the detailed description, which shows and describes illustrative embodiments of the invention. As will be realized, the invention is capable of modifications in various obvious aspects, all without departing from the scope of the present invention as defined in the appended claims.

## Claims

1. A system for delivering a biologic agent and providing an implantable medical device, comprising:
an implantable medical device (10), further referred to as IMD, for providing pacing, cardioversion and/or defibrillation therapy and for monitoring cardiac function;
a reservoir (20) for containing and dispensing a solution containing a biologic;
a lead (30) coupled to the implantable medical device and positionable at a target area within a heart for transmitting signals indicative of cardiac function to the IMD; and
a lumen (50) coupleable to the reservoir and positionable within the lead to allow the solution to be delivered to the target area; **characterized in that** the IMD monitors the efficacy of the biologic agent; and **in that** the IMD provides device based pacing, cardioversion, and/or defibrillation therapy if the efficacy of the biologic agent is below a predetermined threshold.

2. The system of claim 1, wherein the lead is able to transmit signals indicative of a position of the lead within the heart.

3. The system of claim 1, wherein the IMD is able to ablate the targeted area if the efficacy is below a predetermined threshold after a predetermined period of time.

4. The system of claim 1, wherein it is able to deliver a supplemental dose of the biologic to the target area.

5. The system of claim 1, wherein the reservoir is implantable.

6. The system of claim 5, wherein the reservoir is disposed within the IMD.

7. The system of claim 6, wherein the IMD further comprises a self-closing access port (21) in fluid communication with the reservoir, permitting an introduction of material into the reservoir after implantation.

8. The system of claim 4, wherein the IMD further comprises a self-closing access port in fluid communication with the lumen, permitting the supplemental introduction to occur through the lumen.

## Patentansprüche

1. System zum Abgeben eines biologischen Wirkstoffes und Bereitstellen einer implantierbaren medizinischen Vorrichtung, umfassend:
implantierbare medizinische Vorrichtung (10), weiterhin als IMD bezeichnet, zum Bereitstellen einer Schrittmacher-, Kardioversions- und/oder Defibrillationstherapie und zum Überwachen der Herzfunktion;
Behälter (20) zum Aufnehmen und Verabreichen einer Lösung, die ein biologisches Präparat enthält;
Leitung (30), die an die implantierbare medizinische Vorrichtung gekoppelt ist und in einem Zielbereich innerhalb des Herzens platziert werden kann, um Signale, welche die Herzfunktion anzeigen, an die IMD zu übertragen; und
Lumen (50), das an den Behälter gekoppelt und innerhalb der Leitung platziert werden kann, um das Abgeben der Lösung an den Zielbereich zu ermöglichen; **dadurch gekennzeichnet, dass** die IMD die Wirksamkeit des biologischen Wirkstoffes überwacht; und dass die IMD eine auf der Vorrichtung beruhende Schrittmacher-, Kardioversions- und/oder Defibrillationstherapie bereitstellt, wenn die Wirksamkeit des biologischen Wirkstoffes unter einem vorbestimmten Schwellenwert liegt.

2. System nach Anspruch 1, wobei die Leitung in der Lage ist, Signale zu übertragen, die eine Position der Leitung innerhalb des Herzens anzeigen.

3. System nach Anspruch 1, wobei die IMD in der Lage ist, den Zielbereich abzukoppeln, wenn die Wirksamkeit nach einem vorbestimmten Zeitraum unter einem vorbestimmten Schwellenwert liegt.

4. System nach Anspruch 1, wobei es in der Lage ist, eine zusätzliche Dosis des biologischen Präparats an den Zielbereich abzugeben.

5. System nach Anspruch 1, wobei der Behälter implantierbar ist.

6. System nach Anspruch 5, wobei der Behälter innerhalb der IMD angeordnet ist.

7. System nach Anspruch 6, wobei die IMD ferner eine selbstschließende Zutrittsöffnung (21) in Flüssigkommunikation mit dem Behälter umfasst, was eine Einbringung von Material in den Behälter nach der Implantierung erlaubt.

8. System nach Anspruch 4, wobei die IMD ferner eine selbstschließende Zutrittsöffnung in Flüssigkommunikation mit dem Lumen umfasst, was das Stattfinden der zusätzlichen Einbringung durch das Lumen erlaubt.

## Revendications

1. Système pour administrer un agent biologique et fournir un dispositif médical implantable, comprenant :
- un dispositif médical implantable (10), également désigné DMI, pour assurer une thérapie de stimulation électrique, cardioversion et/ou défibrillation et pour surveiller la fonction cardiaque ;
- un réservoir (20) pour contenir et distribuer une solution contenant un agent biologique ;
- un conducteur (30) couplé au dispositif médical implantable et positionnable à une zone cible à l'intérieur d'un coeur pour transmettre des signaux indicatifs de la fonction cardiaque au DMI ; et
- une lumière (50) apte à être couplée au réservoir et positionnable à l'intérieur du conducteur pour permettre à la solution d'être administrée à la zone cible ;
**caractérisé par le fait que** le DMI surveille l'efficacité de l'agent biologique; et **par le fait que** le DMI assure une thérapie de stimulation cardiaque, cardioversion et/ou défibrillation basée sur le dispositif si l'efficacité de l'agent biologique se trouve au-dessous d'un seuil prédéterminé.

2. Système selon la revendication 1, dans lequel le conducteur est capable de transmettre des signaux indicatifs d'une position du conducteur à l'intérieur du coeur.

3. Système selon la revendication 1, dans lequel le DMI est capable de supprimer la zone cible si l'efficacité se trouve au-dessous d'un seuil prédéterminé après une période de temps prédéterminée.

4. Système selon la revendication 1, qui est capable d'administrer une dose supplémentaire de l'agent biologique à la zone cible.

5. Système selon la revendication 1, dans lequel le réservoir est implantable.

6. Système selon la revendication 5, dans lequel le réservoir est disposé à l'intérieur du DMI.

7. Système selon la revendication 6, dans lequel le DMI comprend en outre un orifice d'accès (21) à auto-fermeture en communication de fluide avec le réservoir, permettant une introduction de matière dans le réservoir après implantation.

8. Système selon la revendication 4, dans lequel le DMI comprend en outre un orifice d'accès à auto-fermeture en communication de fluide avec la lumière, permettant à l'introduction supplémentaire de se produire à travers la lumière.
